(19) Europäisches Patentamt / European Patent Office / Office européen des brevets

(11) **EP 1 745 276 B1**

(12) **EUROPEAN PATENT SPECIFICATION**

(45) Date of publication and mention
of the grant of the patent:
**02.09.2015 Bulletin 2015/36**

(21) Application number: **05718514.2**

(22) Date of filing: **18.04.2005**

(51) Int Cl.:
*G01N 17/02* (2006.01)     *G01N 33/38* (2006.01)

(86) International application number:
**PCT/IB2005/001078**

(87) International publication number:
**WO 2005/111575 (24.11.2005 Gazette 2005/47)**

(54) **MEASURING DEVICE, EQUIPMENT AND METHOD FOR MONITORING THE ONSET OF CORROSION AFFECTING STEEL REINFORCEMENTS EMBEDDED IN REINFORCED CONCRETE**

MESSVORRICHTUNG, EINRICHTUNG UND VERFAHREN ZUR ÜBERWACHUNG DES BEGINNS DER KORROSION VON IN STAHLBETON EINGEBETTETEN STAHLTRÄGERN

DISPOSITIF, EQUIPEMENT ET PROCEDE DE MESURE POUR LE CONTROLE DE L'APPARITION DE CORROSION TOUCHANT DES ARMATURES EN ACIER POUR BETON ARME

(84) Designated Contracting States:
**AT BE BG CH CY CZ DE DK EE ES FI FR GB GR HU IE IS IT LI LT LU MC NL PL PT RO SE SI SK TR**

(30) Priority: **14.05.2004 IT MI20040969**

(43) Date of publication of application:
**24.01.2007 Bulletin 2007/04**

(73) Proprietor: **Politecnico Di Milano**
**20133 Milano (IT)**

(72) Inventors:
• **LAZZARI, Luciano**
**I-20131 Milano (IT)**
• **ORMELLESE, Marco**
**I-20131 Milano (IT)**
• **PEDEFERRI, Pietro**
**I-20131 Milano (IT)**

(74) Representative: **Ciceri, Fabio et al**
**Perani & Partners**
**Piazza San Babila, 5**
**20122 Milano (IT)**

(56) References cited:
**WO-A-02/06764     WO-A-96/30741**
**US-A- 5 015 355     US-A- 5 403 550**
**US-A- 6 132 593**

## Description

The present invention relates to a measuring device and a method for monitoring the onset of corrosion affecting steel reinforcements embedded in concrete, particularly but not exclusively corrosion affecting prestressing cables or steel bars embedded in concrete, according to Claims 1 and 13 respectively.

The problem of localized corrosion affecting steel prestressing cables and non-tensioned steel bars forming part of a steel reinforcement embedded in concrete is well known, and has fundamental implications for safety, especially in road engineering works such as motorway bridges or viaducts.

The penetration of chlorides into concrete can lead to the onset of localized corrosion, known as pitting corrosion, which is followed by acidification of the closed cell with the formation of hydrogen in the cavity in which corrosion is taking place. The hydrogen also causes the embrittlement of the steel used for these reinforcements. Because of this process, therefore, whenever corrosion is initiated, this initiation can lead to a catastrophic fracture of the steel cables without any obvious or appreciable warning, with repercussions on safety which can easily be imagined.

There are known methods of measuring the electrochemical potential, in which the onset or propagation of corrosion in reinforced concrete structures exposed to atmospheric agents is monitored by potential mapping. In particular, this mapping is based on the electrochemical mechanism of localized corrosion, in which the anodic corrosion areas, which are small, are separated from the surrounding cathodic areas, with an exchange of electrical current between them. More precisely, the anodic areas are at a more negative potential than the cathodic areas, and the anodic corrosion areas can therefore be identified accurately by measuring the potential at each point, using a grid of suitable size.

For this purpose, there are international standards which can be used to determine whether or not a corrosive phenomenon is occurring in a reinforcing bar in concrete exposed to the atmosphere. Indeed, the interpretation of the data is covered by both international and national standards and regulations, such as ASTM C 876 and UNI 9535.

For example, the ASTM C 876 standard specifies that the areas in which corrosion is occurring show a potential which is more negative than the potential measured by a reference electrode, while areas not subject to corrosion, or passive areas, show a potential which is more positive than that measured by the same reference electrode.

For example, if a copper-copper sulphate reference electrode, commonly known as CSE, is used, it will be found that the areas in which corrosion is occurring show a potential of less than -0.35 V CSE, while areas not subject to corrosion show a potential of more than approximately -0.20 V CSE.

In other words, if the potential measured by the electrode falls below -0.35 V CSE there is a high probability of corrosion in one or more points of the reinforcement.

However, the aforesaid potential mapping method is not applicable to prestressing cables, since these cables are positioned inside a metal or polymer sheath, and are therefore insulated from the surrounding environment.

Furthermore, the technology available at present cannot be used to detect the onset of corrosion in either the initiation or the propagation stage. It is therefore necessary to make use of indirect signal detection, possibly before the start of propagation of the fracture. It should be noted that, for the purposes of safety, it is not necessary to know the exact point at which corrosion has been initiated, but it is essential to know whether or not the corrosion phenomenon has occurred. This is because the cable, or the structure, must be replaced as soon as corrosion occurs.

US Patent 5,403,550 proposes the use of a continuous wire electrode to determine localized corrosion in steel reinforcing cables embedded in concrete.

In particular, it proposes the use of a single wire electrode extending along the longitudinal development of the reinforcing cables (or bars) and in the proximity of the said cables, in order to detect the initiation of corrosion at any point. However, an electrode of this type can supply a signal indicating the onset of a localized attack for certain geometries only, namely for geometries with an "1/D" ratio of less than approximately twenty, where "1" denotes the linear dimension of the wire electrode and of the reinforcing cable, and "D" denotes the thickness of concrete (mortar or cement grout) between the strand and the outer insulating sheath, or, in the case of a bar, between the bar and the outer surface of the concrete exposed to atmospheric agents.

It has also been found experimentally that the potential measured by means of the continuous wire electrode, as described in US patent 5,403,550, is equal to the weighted mean of the potentials of the corresponding surfaces.

This finding can be explained by the fact that the surface of the strands at the point of corrosion (pitting) is much smaller than the rest of the surface, which remains in the passive state. In these conditions, the surface which is the point of corrosion is smaller, in percentage terms, than 0.1% of the total surface area of the strand. The above findings all confirm that the weighted mean of the potentials is practically coincident with the value of the passive surfaces which are not subject to corrosion, since, for real structures, the ratio "1/D" is greater than a thousand on average. This means that the localized corrosion at a given point of the cable is not in fact detectable by measuring the potential with an electrode of this type.

Document US6132593 describes an electrochemical method for measuring localized corrosion and other heterogeneous electrochemical processes. Particular, a multi-sensor electrode namely the wire beam electrode, integrated by coupling all its wire terminals together, is used to simulate a conventional one-piece metal electrode surface in electrochemical behaviour.

Document WO96/30741 describes a combination of electrodes to be moulded into concrete with purpose of measuring environmental parameters in concrete.

In view of the state of the art described above, the object of the present invention is to provide a device which can overcome the limitations of the known art which has been described.

A further object is to provide equipment for monitoring the onset of localized corrosion affecting the steel cables embedded in reinforced concrete. Finally, another object of the present invention is to provide a method for monitoring the onset of localized corrosion affecting the steel cables and bars embedded in reinforced concrete.

According to the present invention, this object is achieved by means of a device for measuring the electrochemical potential of a steel cable or bar in concrete as claimed in Claim 1, and this object is also achieved by means of the method of claim 13.

The present invention makes it possible to carry out continuous monitoring of the localized corrosion conditions affecting steel cables embedded in prestressed reinforced concrete or affecting non-tensioned bars, in other words the reinforcements normally used in bridge slabs, bearing blocks, pillars, reinforced poles, and the like.

The characteristics and advantages of the present invention will be made clear by the following detailed description of a practical embodiment, illustrated by way of example and without restrictive intent in the attached drawings, in which:

- Figure 1 shows a wire electrode for measuring the electrochemical potential according to the present invention;
- Figure 2 shows a measuring device according to the present invention;
- Figure 2A shows a sectional view taken along the line II-II of Figure 2;
- Figure 3 shows a sectional view of a generic prestressing cable according to the prior art;
- Figure 4 shows a schematic view of the measuring electrode of Figure 2 after it has been applied to the strands of a prestressing cable;
- Figure 5 shows a schematic view of equipment for monitoring the localized corrosion according to the present invention;
- Figure 5A shows a sectional view taken along the line III-III of Figure 5;
- Figure 6 shows a schematic view of the measuring electrode of Figure 2 after it has been positioned on the bars of a reinforced concrete structure.

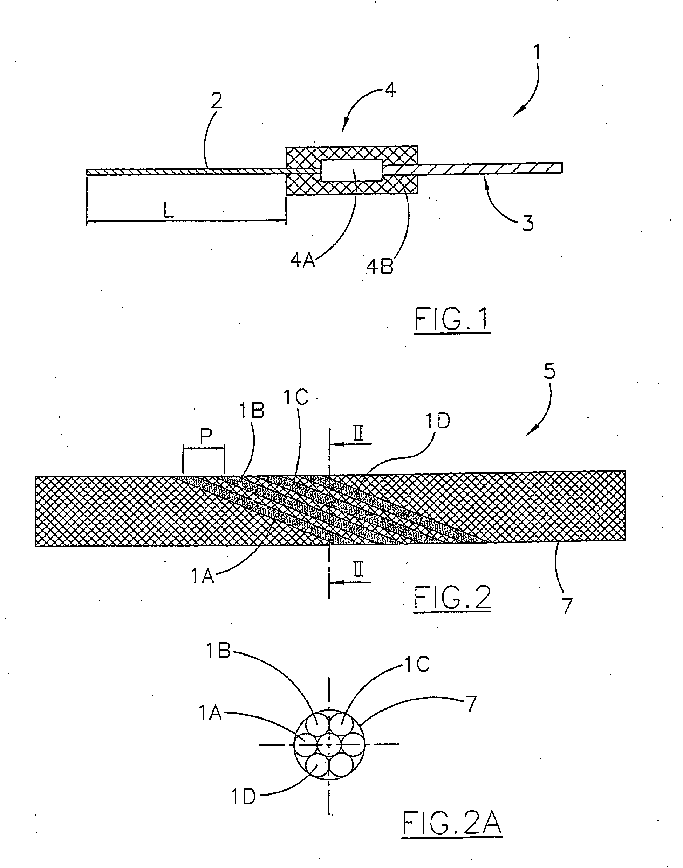

With reference to Figure 1, this shows a wire electrode 1 comprising an active portion 2, associated for operation with a transmission means 3. The active portion 2 of the electrode 1 is the transducer element which can detect a physical magnitude such as potential, while the transmission portion 3 acts as a means of carrying the information obtained by the active portion.

The active portion 2 takes the form of a wire consisting of a metallic material, preferably selected from the group comprising iron, stainless steel, hot-oxidized stainless steel, titanium activated with noble metal oxides, nickel, and copper.

Advantageously, the metallic materials are copper and stainless steel, since these are easily obtainable on the market.

The wire or active portion 2 of the wire electrode 1 has a linear length "L", this length "L" being a function of the specific applications, as described below.

The transmission means 3 may, for example, take the form of an electrical cable.

For example, if copper is selected as the metallic material to form the active portion 2, the cable 3 can be ordinary commercially available electrical cable, used for constructing electrical installations for public and/or industrial use.

The junction between the active portion 2 and the transmission means 3 is made by processes known to those skilled in the art; alternatively, according to an optional aspect of the present invention, the active portion 2 and the transmission means 3 are connected to each other by connecting means 4.

These connecting means 4 connect the active part 2 electrically to the transmission part 3, while also acting means of insulation from the concrete. This prevents the concrete from penetrating into the junction area between the wire 2 and the electrical cable 3.

These connecting means 4 may, for example, take the form of a heat-shrinking sheath 4A, acting as the electrical connection element, wrapped with a sealing resin 4B which acts as the insulating element.

A measuring device 5 according to the present invention is shown in Figure 2, which shows that the said measuring device 5 comprises a plurality of wire electrodes 1, indicated respectively by 1A, 1B, 1C, etc., each of which consists of its own electrical connecting cable 3 and active portion 2.

In turn, this measuring device 5 is contained (or wrapped) in an insulating sheath 7 which can resist the alkalinity of the concrete.

The sheath 7 is made, for example, from a perforated plastics material.

The measuring device 5 thus resembles a single multistrand cable.

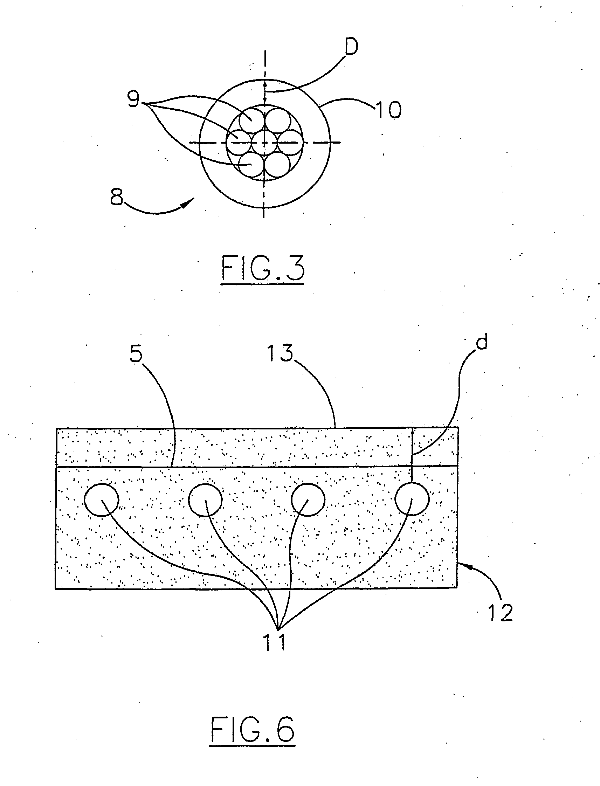

Figure 3 shows a section through a prestressing cable 8 forming part of a steel reinforcement. The cable 8 is formed, for example, from a core consisting of a plurality of strands 9, the latter being contained (or wrapped) in a sheath 10. The strands 9 can be, for example, interleaved and wound together in spiral form.

Between the sheath 10 and the strands 9 there is a gap (or interstice) "D", called the concrete cover. This space "D" is filled with alkaline mortar or cement grout (which acts as an electrolyte).

The measuring device 5 has to be inserted into the gap formed by the space "D".

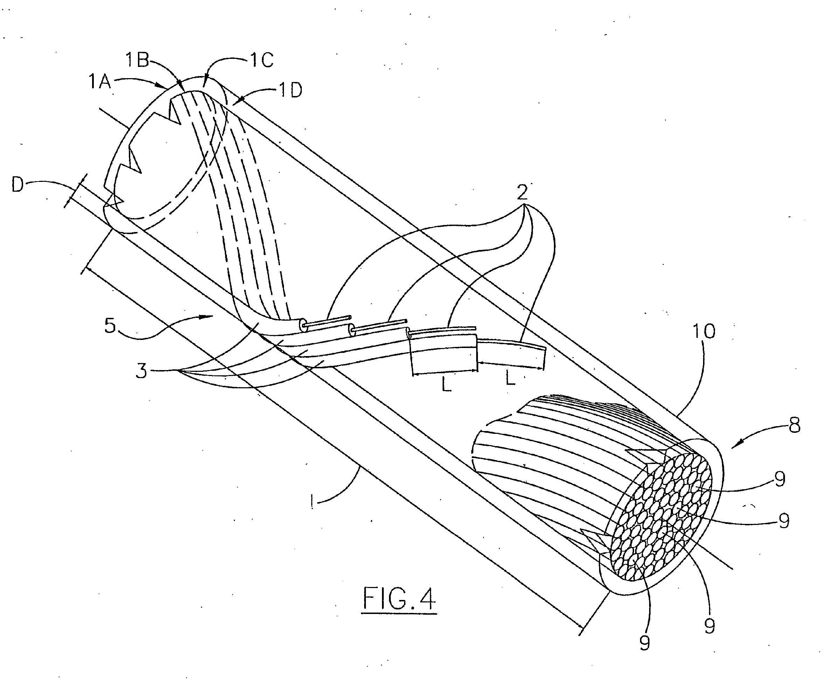

Moving on to Figure 4, it will be noted that the measuring device 5 is fitted on top of the strands 9 of a prestressing cable 8. This measuring device 5 can be positioned in the proximity of the said strands 9 and/or directly associated with the said strands 9.

In particular, it should be noted that Figure 4 shows a possible arrangement of the wire electrodes 1A, 1B, 1C, 1D along the strands 9 in the case in which there is a direct association between the measuring device 5 and the strands 9 of the cable 8.

The wire electrodes 1A, 1B, 1C and 1D, forming the measuring device 5, are positioned in the concrete cover D, in other words in the gap D formed between the strands 9 and the sheath 10.

It should also be noted that the wire electrodes 1A, 1B, 1C and 1D are positioned so as to cover a portion of the linear length "1" of the strands 9. It is also possible for the wire electrodes 1A, 1B, 1C and 1D to be positioned so as to cover the whole of the linear length "1" of the strands 9.

More precisely, it should be noted that the active portions 2 of each wire electrode are positioned in series with each other. In other words, the terminal part (the tail) of the active portion 2 of the first wire electrode 1A terminates at the start of the leading part (the head) of the active portion of the second wire electrode 1B, and so on. This results in a "head-to-tail" arrangement.

In the specific embodiment shown in Figure 4, the first wire electrode 1A has the function of monitoring a first portion of the cable 8, the second wire electrode 1B has the function of measuring a second portion of the cable, and so on, the first and second portions each having an equal length "L".

In another embodiment, not shown in the figures, it is also possible for the first wire electrode 1A to have the function of monitoring one portion of the cable 8, while the second wire electrode 1B has the function of measuring another portion of the cable 8, without these portions being consecutive with respect to each other.

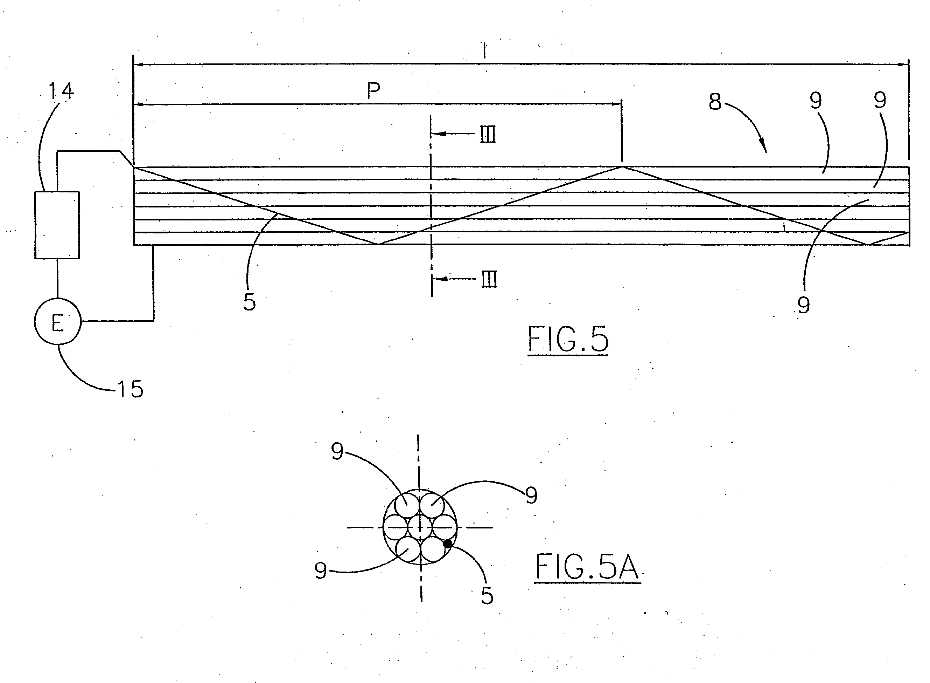

With reference to Figure 5, it may be noted the measuring device 5 is wound in a spiral with a suitable pitch "P" around the strands 9 of the cable 8. The pitch "P" of the winding of the said device 5 around the strands 9 is a function of the specific application and is, for example, in the range from one to three metres, or is preferably equal to two metres.

In another embodiment, not shown in the figures, the measuring device 5 can be extended parallel to the strands.

With particular reference to Figure 6, what is shown here is another possible application of the measuring device 5. In Figure 6, it can be seen that the measuring device 5 is installed in the proximity of the bars 11 of a non-tensioned structure 12 and an outer surface 13. For example, the non-tensioned structure 12 could be of the type used in the slabs of bridges, bearing blocks, pillars, reinforced poles, and the like.

In particular, the bars 11 of the non-tensioned structure 12 are embedded in the concrete at a distance "d" from the outer surface 13.

In this embodiment also, the measuring device 5 is to be positioned in this thickness "d". It should be noted that the thickness "d" is also usually called the "concrete cover".

In the specific application described in Figure 6, the measuring device 5 is again installed according to the previous description given with reference to Figure 4.

The ratio between the length "L" of the active portion 2 of each wire electrode 1A, 1B, 1C, etc. and the concrete cover "D" (or "d") is of fundamental importance for the identification of the onset of localized corrosion along the strands 9 of the cable 8 or along the bars 11 of the non-tensioned structure 12.

Thus the ratio "L/D" (or "L/d") can be used to determine the most suitable geometry for monitoring the strands 9 or bars 11 of any steel reinforcement embedded in the concrete.

In particular, the interval "L/D", according to the present invention, is in the range from zero to thirty, in other words:

$$L/D = 0 \div 30$$

or preferably $15 \div 20$.

In other words, given the length "1" of the strands 9 of the cable 8 and the available concrete cover "D", this ratio L/D can be used to find the requisite linear length "L" of the active portion 2 of each wire electrode 1A, 1B, 1C, etc. making up the measuring device 5, and the number of steel wires making up the said device.

For example, if the onset of corrosion is to be monitored on strands 9 with a length "l" equal to eighteen metres, and the concrete cover "D" is equal to one centimetre, then, in the case in which the ratio "L/D" is assumed to be thirty, we find that the length "L" of each active portion 2 of the wire electrode must be thirty centimetres.

Thus, in order to cover the whole length "1" of the cable 8, it is necessary to provide sixty individual wire electrodes 1A, 1B, etc., each of which must be provided with both an active portion 2 and a corresponding electrical signal transmission cable 3. A similar reasoning can be applied to the case of the bars 11 of the concrete structure 12. This is because, given the length "l" of the bars 11 and the available concrete cover "d", the ratio "L/d" can be used to find the requisite linear length "L" of the active portion 2 of each wire electrode 1A, 1B, 1C, etc. making up the measuring device 5, and the number of steel wires making up the said device.

As mentioned above, the first stage of the corrosion phenomenon is the formation of a first corrosion cell (a pit), at any point of a steel reinforcement, regardless of whether this consists of a cable 8 or of the bars 11 of the structure 12.

The appearance of the pit can be detected in real time by the "local" measurement of the electrochemical corrosion potential of the strand 9 or the bar 11, in other words by a measurement made in the immediate vicinity of the corroded area itself, exploiting the presence of the active portion 2 of each wire electrode 1A, 1B, 1C, etc.

Referring to Figures 5 and 5A, which show equipment for measuring the electrochemical potential in the cable 8, it can be seen that the measuring device 5 is wound in a spiral with a pitch "P" on the strands 9 along the whole linear length "l" of the cable 8. In particular, each wire electrode 1A, 1B, 1C, etc. forming the measuring device 5 is connected to a junction box or data acquisition card 14. In turn, this data acquisition card 14 is connected to a potential measuring instrument 15, for example a high-impedance voltmeter.

Specifically, the transmission cables 3 of each wire 2 are connected to the data acquisition card 14. This card 14 also has the function of housing one or more devices (not shown in the figures) for carrying out processing operations on the measurements made by each wire electrode and/or for storing these measurements and/or the data extrapolated from them. These devices can, for example, take the form of non-volatile memories and processors.

The voltmeter 15 has the cable 8 as its positive terminal, while its negative terminal is the acquisition card 14, in other words the transmission cables 3 of each active portion 2.

If "i" denotes the number of wire electrodes 1A, 1B, 1C, etc. used to monitor the strands 9 or a portion of the strands, then when one or more of the said wire electrodes 1A, 1B, 1C, etc. is in the vicinity of an area in which the corrosion phenomenon is occurring, each of the said wire electrodes 1A, 1B, 1C, etc. detects its own potential difference $\Delta V_i$.

If even a single measurement of the potential difference $\Delta V_i$ is more negative than the preceding measurement of the same wire electrode, and/or more negative than the mean of the other wire electrodes, by a predetermined threshold value $\xi$, then corrosion has been initiated at this point of the strand.

It should be noted that the mean can be calculated on the basis of a measurement made in real time by all the wire electrodes 1A, 1B, 1C, etc., or by taking a historic mean of the measurements made previously by these wire electrodes 1A, 1B, 1C, etc., or by a combination of these methods.

If one or more of the wire electrodes 1A, 1B, 1C, etc. supplies a potential measurement below the predetermined threshold value $\xi$, for example less than at least one hundred millivolts, and in any case below the preceding mean of the said threshold value $\xi$, in other words at least one hundred millivolts, this means that the phenomenon of localized corrosion is occurring in the area to which the wire electrode relates.

According to the present invention, the method by which the potential difference $\Delta V_i$ is measured is as follows:

- a potential difference $\Delta V_i$ is measured for each "i", where "i" is the number of wire electrodes used to monitor the strands 9 of the cable 8 or the bars 11 of the non-tensioned structure 12;
- a statistical analysis is performed on the measured potentials $\Delta V_i$;
- the measurement of the potential difference $\Delta V_i$ is repeated with a frequency T specified in advance; in other words, the potentials $\Delta V_i$ are measured at the time T=1 for each "i", the measurement of the potentials $\Delta V_i$ are repeated at the time T=2 for each "i", the potentials $\Delta V_i$ are repeated at the time T=3 for each "i", and so on;
- if necessary, a step of storing the said measured data, namely the value of the potential difference $\Delta V_i$ at the time T=1 for each "i", the value of the potential difference $\Delta V_i$ at the time T=2 for each "i", the value of the potential difference $AV_i$ at the time T=3 for each "i", and so on, is carried out;
- each measurement is compared with the preceding result and with the mean $\mu$ of the preceding measurements, in other words $\Delta V_i$ at the time T=3 is compared with the measurement of $\Delta V_i$ at the time T=2 and with the mean value $\mu$ of the measurements of $\Delta V_i$ at the time T=1 and at the time T=2;
- each measurement is compared with the value of the variance $\sigma$ of each preceding measure, and finally
- a signal is generated when the measurement of the said potential $\Delta V_i$ of only one of the said wire electrodes 1A, 1B, 1C, etc. yields a value which is lower than the preceding value by at least a predetermined value $\xi$.

The frequency T of repetition of the measurement is a function of the specific application, and can vary from minutes to years. Preferably, the frequency of measurement T is set at one week.

In particular, the statistical analysis of the measured values is carried out by determining the minimum and/or maximum and/or mean value of each measurement made and the value of the variance $\sigma$ of each measurement.

Thus the onset of localized corrosion in the strands 9 of prestressing cables 8, or in the bars 11 of reinforcing structures 12, embedded in concrete can be detected in good time by continual measurement of the potential $\Delta V_i$ between the measuring device 5 and the cable 8 or the bar 11 of the non-tensioned structure 12.

**Claims**

1. Reinforced concrete, comprising a device (5) for measuring the electrochemical potential of a steel reinforcement (8, 11) embedded in concrete, the said measuring device (5) being capable of being associated with the said steel reinforcement (8, 11), said device comprising a plurality of wire electrodes (1A, 1B, 1C, 1D), each having an active portion (2) for detecting an electrochemical potential ($\Delta V_i$) and being capable of being associated for operation with a transmission means (3) in such a way that the said detected electrochemical potential ($\Delta V_i$) can be transmitted, in order to make a local measurement of the onset of corrosion in the said reinforcement (8, 11), **characterized in that** each active portion (2) has a linear length (L) in a range defined by the ratio between a concrete cover (D) and a linear length (l) of the said steel reinforcement (8,11), so as to cover a portion or the whole of the linear length (1) of the said steel reinforcement (8,11), said wire electrodes being positioned in the concrete cover (D).

2. Reinforced concrete according to Claim 1, **characterized in that** said concrete cover (D) being the distance between a sheath (10) enclosing a plurality of strands (9) of the said cable (8) and the said plurality of strands (9) themselves, said range varying from zero to thirty, preferably between 15 ÷ 20.

3. Reinforced concrete according to Claim 1, **characterized in that** said concrete cover (d) being the distance between the said bar (11) and a surface (12) exposed to atmospheric agents, said range varying from zero to thirty, preferably between 15 ÷ 20.

4. Reinforced concrete according to Claim 1, **characterized in that** the said active portions (2) of each wire electrode are positioned in series with each other.

5. Reinforced concrete according to Claim 4, **characterized in that** the said metallic wire comprises one of the following materials: iron, stainless steel, hot-oxidized stainless steel, titanium activated with noble metal oxides, nickel, and copper.

6. Reinforced concrete according to Claim 1, **characterized in that** the said plurality of wire electrodes (1A, 1B, 1C, 1D) are wound together in spiral form.

7. Reinforced concrete according to Claim 1, **characterized in that** the said active portion (2) is connected to the said transmission means (3) by connecting means (4).

8. Reinforced concrete according to Claim 7, **characterized in that** the said connecting means (4) comprise an electrical connecting sheath (4A) wrapped in a sealing resin (4B) acting as an insulating element.

9. Reinforced concrete according to Claim 1, **characterized in that** the said measuring device (5) is wrapped in a perforated insulating sheath (7).

10. Reinforced concrete according to Claim 9, **characterized in that** the said perforated insulating sheath (7) is made from plastics material resistant to the alkalinity of the concrete.

11. Reinforced concrete according to Claim 1, **characterized in that** the said transmission means (3) is an electrical cable.

12. Reinforced concrete according to Claim 1, comprising reading means (15) for reading the said electrochemical potential ($\Delta V_i$) between each active portion (2) of the said plurality of wire electrodes (1A, 1B, 1C, 1D) and the said steel reinforcement (8, 11) and an acquisition card (14) connected on the one hand to the said measurement means (15) and on the other hand to the transmission portion (3) of the said measuring device (5).

13. A method for measuring the electrochemical potential of a steel reinforcement (8, 11) embedded in concrete, comprising a measuring device (5) comprising a plurality of wire electrodes (1A, 1B, 1C, 1D), each having an active portion (2) for detecting the said electrochemical potential ($\Delta V_i$), each active portion (2) having a linear length (L) in a range defined by the ratio between a concrete cover (D,d) and a linear length (L,l) of the said steel reinforcement (8,11), each active portion (2) being associated for operation with a transmission means (3) in such a way as to transmit the said detected electrochemical potential ($\Delta V_i$), **characterized in that** it comprises the steps of:

- measuring a potential difference ($\Delta V_i$) between at least one of the said active portions (3) of the said wire electrodes (1A, 1B, 1C, 1D) and the said steel reinforcement (8, 11);
- repeating the said measurement of potential ($\Delta V_i$) at a predetermined frequency (T);
- comparing each potential measurement ($\Delta V_i$) with the preceding result and with the mean ($\mu$) of the preceding measurements;
- generating a signal when the measurement of the said potential ($\Delta V_i$) of only one of the said wire electrodes (1A, 1B, 1C, etc.) yields a value which is lower than the preceding value by at least a predetermined value ($\xi$), wherein said wire electrodes are positioned in the concrete cover (D)

14. A method according to Claim 13, **characterized in that** it additionally comprises the step of:

   - comparing each potential measurement ($\Delta V_i$) with the variances ($\sigma$) of the said measurements.

15. A method according to Claim 13, **characterized in that** the said predetermined frequency (T) is in the range from minutes to years.

16. A method according to Claim 13, comprising the step of positioning in sequence with each other along at least a portion of the said reinforcement (8, 11) said plurality of wire electrodes (1A, 1B, 1C, 1D), in such a way that each active portion (2) detects the said electrochemical potential ($\Delta V_i$).

17. A method according to Claim 13, comprising the step of wounding around said reinforcement (8, 11) with a predetermined pitch (P) said plurality of wire electrodes (1A, 1B, 1C, 1D).

18. A method according to Claim 17, wherein said pitch (P) is in,the range from one to three metres.

19. A method according to Claim 13, comprising the step of positioning parallel to the said reinforcement (8, 11) said plurality of wire electrodes (1A, 1B, 1C, 1D).

**Patentansprüche**

1. Stahlbeton, der eine Vorrichtung (5) zum Messen des elektrochemischen Potentials einer in Beton eingebetteten Stahlbewehrung (8, 11) umfasst, wobei diese Messeinrichtung (5) mit der Stahlbewehrung (8, 11) verbunden sein kann, wobei diese Vorrichtung eine Vielzahl von Drahtelektroden (1A, 1B, 1C, 1D) umfasst, die jeweils einen aktiven Abschnitt (2) zum Detektieren eines elektrochemischen Potentials ($\Delta V_i$) aufweisen und für den Betrieb derart mit einem Übertragungsmittel (3) verbunden sein können, dass das detektierte elektrochemische Potential ($\Delta V_i$) übertragen werden kann, um eine lokale Messung des Beginns von Korrosion in der Bewehrung (8, 11) durchzuführen, **dadurch gekennzeichnet, dass** jeder aktive Abschnitt (2) eine lineare Länge (L) in einem Bereich aufweist, der durch das Verhältnis zwischen einer Betonüberdeckung (D) und einer linearen Länge (1) der Stahlbewehrung (8, 11) definiert ist, so dass ein Teil oder das Ganze der linearen Länge (1) der Stahlbewehrung (8, 11) abgedeckt ist, wobei die Drahtelektroden in der Betonüberdeckung (D) angeordnet sind.

2. Stahlbeton nach Anspruch 1, **dadurch gekennzeichnet, dass** die Betonüberdeckung (D) der Abstand zwischen einem Hüllrohr (10), das eine Vielzahl von Drähten (9) des Kabels (8) umschließt, und dieser Vielzahl von Drähten (9) selbst ist, wobei der Bereich von null bis dreißig, vorzugsweise zwischen 15 und 20, variiert.

3. Stahlbeton nach Anspruch 1, **dadurch gekennzeichnet, dass** die Betonüberdeckung (d) der Abstand zwischen der Stange (11) und einer den Witterungseinflüssen ausgesetzten Oberfläche (12) ist, wobei der Bereich von null bis dreißig, vorzugsweise zwischen 15 und 20, variiert.

4. Stahlbeton nach Anspruch 1, **dadurch gekennzeichnet, dass** die aktiven Abschnitte (2) von jeder Drahtelektrode in Reihe miteinander angeordnet sind.

5. Stahlbeton nach Anspruch 4, **dadurch gekennzeichnet, dass** der Metalldraht eines der folgenden Materialien umfasst: Eisen, rostfreier Stahl, unter Hitze oxidierter rostfreier Stahl, mit Edelmetalloxiden aktiviertes Titan, Nickel und Kupfer.

6. Stahlbeton nach Anspruch 1, **dadurch gekennzeichnet, dass** die Vielzahl von Drahtelektroden (1A, 1B, 1C, 1D) spiralförmig gemeinsam aufgewickelt sind.

7. Stahlbeton nach Anspruch 1, **dadurch gekennzeichnet, dass** der aktive Abschnitt (2) durch Verbindungsmittel (4) mit dem Übertragungsmittel (3) verbunden ist.

8. Stahlbeton nach Anspruch 7, **dadurch gekennzeichnet, dass** die Verbindungsmittel (4) eine elektrische Verbindungshülle (4A) umfassen, die in ein Dichtungsharz (4B) eingehüllt ist, das als Isolierelement dient.

9. Stahlbeton nach Anspruch 1, **dadurch gekennzeichnet, dass** die Messeinrichtung (5) in eine perforierte Isolierhülle (7) eingehüllt ist.

10. Stahlbeton nach Anspruch 9, **dadurch gekennzeichnet, dass** die perforierte Isolierhülle (7) aus Kunststoffmaterial besteht, das resistent gegen die Alkalinität des Betons ist.

11. Stahlbeton nach Anspruch 1, **dadurch gekennzeichnet, dass** das Übertragungsmittel (3) ein elektrisches Kabel ist.

12. Stahlbeton nach Anspruch 1, der Messmittel (15) zum Messen des elektrochemischen Potentials ($\Delta V_i$) zwischen jedem aktiven Abschnitt (2) der Vielzahl von Drahtelektroden (1A, 1B, 1C, 1D) und der Stahlbewehrung (8, 11) und eine Erfassungskarte (14) umfasst, die einerseits mit diesen Messmitteln (15) und andererseits mit dem Übertragungsabschnitt (3) der Messeinrichtung (5) verbunden ist.

13. Verfahren zum Messen des elektrochemischen Potentials einer in Beton eingebetteten Stahlbewehrung (8, 11), das eine Messeinrichtung (5) umfasst, die eine Vielzahl von Drahtelektroden (1A, 1B, 1C, 1D) umfasst, die jeweils einen aktiven Abschnitt (2) zum Detektieren des elektrochemischen Potentials ($\Delta V_i$) aufweisen, wobei jeder aktive Abschnitt (2) eine lineare Länge (L) in einem Bereich aufweist, der durch das Verhältnis zwischen einer Betonüberdeckung (D, d) und einer linearen Länge (L, l) der Stahlbewehrung (8, 11) definiert ist, wobei jeder aktive Abschnitt (2) für den Betrieb derart mit einem Übertragungsmittel (3) verbunden ist, dass er das detektierte elektrochemische Potential ($\Delta V_i$) überträgt, **dadurch gekennzeichnet, dass** es die folgenden Schritte umfasst:

- Messen einer Potentialdifferenz ($\Delta V_i$) zwischen mindestens einem der aktiven Abschnitte (3) der Drahtelektroden (1A, 1B, 1C, 1D) und der Stahlbewehrung (8, 11);
- Wiederholen dieser Potentialmessung ($\Delta V_i$) mit einer vorbestimmten Frequenz (T);
- Vergleichen jeder Potentialmessung ($\Delta V_i$) mit dem vorhergehenden Ergebnis und mit dem Mittelwert ($\mu$) der vorhergehenden Messungen;
- Erzeugen eines Signals, wenn die Messung des Potentials ($\Delta V_i$) von nur einer der Drahtelektroden (1A, 1B, 1C usw.) einen Wert ergibt, der um mindestens einen vorbestimmten Wert ($\xi$) niedriger als der vorhergehende Wert ist, wobei die Drahtelektroden in der Betonüberdeckung (D) angeordnet sind.

14. Verfahren nach Anspruch 13, **dadurch gekennzeichnet, dass** es zusätzlich den folgenden Schritt umfasst:

- Vergleichen jeder Potentialmessung ($\Delta V_i$) mit den Varianzen ($\sigma$) der Messungen.

15. Verfahren nach Anspruch 13, **dadurch gekennzeichnet, dass** die vorbestimmte Frequenz (T) in einem Bereich von Minuten bis Jahren liegt.

16. Verfahren nach Anspruch 13, das den Schritt des Anordnens der Vielzahl von Drahtelektroden (1A, 1B, 1C, 1D) in Reihe miteinander entlang zumindest einem Abschnitt der Bewehrung (8, 11) derart umfasst, dass jeder aktive Abschnitt (2) das elektroche-

mische Potential ($\Delta V_i$) detektiert.

17. Verfahren nach Anspruch 13, das den Schritt des Wickelns der Vielzahl von Drahtelektroden (1A, 1B, 1C, 1D) um die Bewehrung (8, 11) mit einer vorbestimmten Steigung (P) umfasst.

18. Verfahren nach Anspruch 17, wobei die Steigung (P) im Bereich von einem bis drei Metern liegt.

19. Verfahren nach Anspruch 13, das den Schritt des Anordnens der Vielzahl von Drahtelektroden (1A, 1B, 1C, 1D) parallel zur Bewehrung (8, 11) umfasst.

**Revendications**

1. Béton armé, comprenant un dispositif (5) pour mesurer le potentiel électrochimique d'une armature en acier (8, 11) noyée dans le béton, ledit dispositif de mesure (5) étant adapté pour être associé à ladite armature en acier (8, 11), ledit dispositif comprenant une pluralité de fils-électrodes (1A, 1B, 1C, 1D), chacun ayant une portion active (2) pour détecter un potentiel électrochimique ($\Delta V_i$) et étant adaptée pour être associée pour le fonctionnement à un moyen de transmission (3) de telle manière que ledit potentiel électrochimique détecté ($\Delta V_i$) puisse être transmis, de manière à effectuer une mesure locale de l'apparition de corrosion dans ladite armature (8, 11), **caractérisé en ce que** chaque portion active (2) a une longueur linéaire (L) dans une plage définie par le rapport entre une couverture de béton (D) et une longueur linéaire (l) de ladite armature en acier (8, 11), de manière à couvrir une portion ou la totalité de la longueur linéaire (1) de ladite armature en acier (8, 11), lesdits fils-électrodes étant positionnés dans la couverture de béton (D).

2. Béton armé selon la revendication 1, **caractérisé en ce que** ladite couverture de béton (D) est la distance entre une gaine (10) enfermant une pluralité de brins (9) dudit câble (8) et ladite pluralité de brins (9) elle-même, ladite plage variant de zéro à trente, de préférence entre 15 et 20.

3. Béton armé selon la revendication 1, **caractérisé en ce que** ladite couverture de béton (d) est la distance entre ladite barre (11) et une surface (12) exposée à des agents atmosphériques, ladite plage variant de zéro à trente, de préférence entre 15 et 20.

4. Béton armé selon la revendication 1, **caractérisé en ce que** lesdites portions actives (2) de chaque fil-électrode sont positionnées en série l'une par rapport à l'autre.

5. Béton armé selon la revendication 4, **caractérisé en**

**ce que** ledit fil métallique comprend un des matériaux suivants : fer, acier inoxydable, acier inoxydable oxydé à chaud, titane activé avec des oxydes de métaux nobles, nickel et cuivre.

6. Béton armé selon la revendication 1, **caractérisé en ce que** ladite pluralité de fils-électrodes (1A, 1B, 1C, 1D) est enroulée conjointement en forme de spirale.

7. Béton armé selon la revendication 1, **caractérisé en ce que** ladite portion active (2) est connectée audit moyen de transmission (3) par des moyens de connexion (4).

8. Béton armé selon la revendication 7, **caractérisé en ce que** lesdits moyens de connexion (4) comprennent une gaine de connexion électrique (4A) enveloppée dans une résine de scellement (4B) agissant comme un élément isolant.

9. Béton armé selon la revendication 1, **caractérisé en ce que** ledit dispositif de mesure (5) est enveloppé dans une gaine isolante perforée (7).

10. Béton armé selon la revendication 9, **caractérisé en ce que** ladite gaine isolante perforée (7) est réalisée en matériau plastique résistant à l'alcalinité du béton.

11. Béton armé selon la revendication 1, **caractérisé en ce que** ledit moyen de transmission (3) est un câble électrique.

12. Béton armé selon la revendication 1, comprenant des moyens de lecture (15) pour lire ledit potentiel électrochimique ($\Delta V_i$) entre chaque portion active (2) de ladite pluralité de fils-électrodes (1A, 1B, 1C, 1D) et de ladite armature en acier (8, 11) et une carte d'acquisition (14) connectée d'une part auxdits moyens de mesure (15) et d'autre part à la portion de transmission (3) dudit dispositif de mesure (5).

13. Procédé pour mesurer le potentiel électrochimique d'une armature en acier (8, 11) noyée dans le béton, comprenant un dispositif de mesure (5) comprenant une pluralité de fils-électrodes (1A, 1B, 1C, 1D), chacun ayant une portion active (2) pour détecter ledit potentiel électrochimique ($\Delta V_i$), chaque portion active (2) ayant une longueur linéaire (L) dans une plage définie par le rapport entre une couverture de béton (D, d) et une longueur linéaire (L, 1) de ladite armature en acier (8, 11), chaque portion active (2) étant associée pour le fonctionnement à un moyen de transmission (3) de manière à transmettre ledit potentiel électrochimique détecté ($\Delta V_i$), **caractérisé en ce qu'**il comprend les étapes suivantes :

   - la mesure d'une différence de potentiel ($\Delta V_i$) entre au moins une desdites portions actives (3) desdits fils-électrodes (1A, 1B, 1C, 1D) et ladite armature en acier (8, 11) ;
   - la répétition de ladite mesure de potentiel ($\Delta V_i$) à une fréquence prédéterminée (T) ;
   - la comparaison de chaque mesure de potentiel ($\Delta V_i$) avec un résultat précédent et avec la moyenne ($\mu$) des mesures précédentes ;
   - la génération d'un signal quand la mesure dudit potentiel ($\Delta V_i$) d'un seul desdits fils-électrodes (1A, 1B, 1C, etc.) produit une valeur qui est inférieure à la valeur précédente d'au moins une valeur prédéterminée ($\xi$), dans lequel lesdits fils-électrodes sont positionnés dans la couverture de béton (D).

14. Procédé selon la revendication 13, **caractérisé en ce qu'**il comprend en plus l'étape suivante :

   - la comparaison de chaque mesure de potentiel ($\Delta V_i$) avec les variances ($\sigma$) desdites mesures.

15. Procédé selon la revendication 13, **caractérisé en ce que** ladite fréquence prédéterminée (T) est dans la plage de minutes à années.

16. Procédé selon la revendication 13, comprenant l'étape de positionnement en séquence l'un par rapport à l'autre le long d'au moins une portion de ladite armature (8, 11) de ladite pluralité de fils-électrodes (1A, 1B, 1C, 1D), de telle manière que chaque portion active (2) détecte ledit potentiel électrochimique ($\Delta V_i$).

17. Procédé selon la revendication 13, comprenant l'étape d'enroulement autour de ladite armature (8, 11) avec un pas prédéterminé (P) de ladite pluralité de fils-électrodes (1A, 1B, 1C, 1D).

18. Procédé selon la revendication 17, dans lequel ledit pas (P) est dans la plage de un à trois mètres.

19. Procédé selon la revendication 13, comprenant l'étape de positionnement parallèle à ladite armature (8, 11) de ladite pluralité de fils-électrodes (1A, 1B, 1C, 1D).

FIG.1

FIG.2

FIG.2A

D

9

10

8

## FIG.3

5    13    d

11

12

## FIG.6

FIG.4

EP 1 745 276 B1

FIG.5

FIG.5A

EP 1 745 276 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5403550 A
- US 6132593 A
- WO 9630741 A